# EUROPEAN PATENT APPLICATION

(11) **EP 1 679 068 A1**
(43) Date of publication of application: **12.07.2006**
(21) Application number: 04793331.2
(22) Date of filing: 28.10.2004
(51) Int. Cl.: A61K 31/202, A61K 31/232, A61K 31/66, A61P 9/10, A61P 9/00, A23L 1/30

(54) **COMPOSITION FUNCTIONING TO PREVENT OR MITIGATE SYMPTOM OR DISEASE ATTRIBUTABLE TO BLOOD VESSEL AGING**

(30) Priority: 29.10.2003 JP 2003369147
(71) Applicant: SUNTORY LIMITED, Osaka-shi, Osaka 530-8203 (JP)
(72) Inventor: ISHIKURA, Yoshiyuki, Ibaraki-shi, Osaka 5670874 (JP); HORIKAWA, Chika, Ibaraki-shi, Osaka 5670868 (JP); ONO, Yoshiko, Osaka-shi, Osaka 5330022 (JP); AKIMOTO, Kengo, Kawasaki-shi, Kanagawa 2110036 (JP); MATSUMURA, Yasuo, Kitakatsuragi-gun, Nara 6392142 (JP)
(74) Representative: Stoner, Gerard Patrick
(86) International application number: PCT/JP2004/016351
(87) International publication number: WO 2005/039559

(57) **Abstract**

A composition and food or beverage containing arachidonic acid or a compound having arachidonic acid as a component fatty acid and having an action preventing or alleviating symptoms or diseases due to aging of the blood vessels.

## Description

### BACKGROUND OF THE INVENTION

### 1. Field of the Invention

The present invention relates to a composition having arachidonic acid or a compound having arachidonic acid as a component fatty acid as an active ingredient and a food or beverage having an action preventing or alleviating symptoms or diseases due to aging of the blood vessels and a method of production of the same. More specifically, the invention relates to a composition having at least one of arachidonic acid, an alcohol ester of an arachidonic acid, a triglyceride having arachidonic acid as a component fatty acid, and a phospholipid as an active ingredient and having an action preventing or alleviating a drop in elasticity of the blood vessels, arteriosclerosis (for example, ischemic cardiac diseases (myocardial infarction and angina), or cerebral apoplexy (cerebral hemorrhage and cerebral infarction)), a food or beverage having a preventative or alleviating action, and a method of production of the same.

### 2. Description of the Related Art

In recent years, progress in medicine has led to a rapid increase in the proportion of senior citizens in society. Along with this, the number of patients suffering from arteriosclerosis has been increasing. According to the *FY2000 Health and Welfare White Paper* and the *Report on Studies of Measures for Senior Citizens Suffering From Cerebral Infarction* of Japan, there were 1.5 to 1.6 million patients suffering from arteriosclerosis in that country alone in FY2000. One out of every 14 Japanese senior citizens aged 65 or more suffers from arteriosclerosis. It is expected that the number of patients may certainly increase to one per 10 persons in 2030. Arteriosclerosis may be roughly divided into pultaceous arteriosclerosis, media calcification and hardening, and arteriocapillary sclerosis.

Pultaceous arteriosclerosis is clinically important. Prime starting locations are from the major arteries to the arteries of the four limbs, the coronary artery, the anterior cerebral artery, etc. If arteriosclerosis progresses, symptoms of ischemic cardiac diseases, cerebral infarction, including carotid artery constriction, and arteriosclerosis obliterans become observed and daily life or social activities become hampered. Risk factors of pultaceous arteriosclerosis include, in addition to high cholesterol, aging, high blood pressure, diabetes, smoking, family history of coronary arterial disease, low HDL-cholesterol, etc., but these are believed to be the result of the combination of various factors promoting pultaceous arteriosclerosis (*Yakkyoku (Pharmacology)* 54, 2245-2249, 2003).

From this thinking, arteriosclerosis type diseases have been generally treated by primary preventative treatment by hyperlipemia drugs, high blood pressure drugs, diabetic drugs, etc. in accordance with the presumed risk factors and by secondary preventative treatment by anti-platelet drugs, ACE inhibitors, β-blockers, anti-coagulants, brain-protecting agents, etc. in accordance with the symptoms of the ischemic cardiac diseases or cerebral infarction *(Yakkyoku (Pharmacology)* 54, 2287-2303, 2003). However, almost all of these are methods of treatment of symptoms such as by anti-platelet coagulants and anti-thrombin drugs for suppressing formation of platelets in the brain, brain-protecting agents for suppressing cell damage in cerebral ischemia, drugs for hyperlipemia, the risk factor for arteriosclerosis, and depressors for high blood pressure. No drug for alleviating changes along with aging of the blood vessels has been known at all.

Arteriosclerosis type diseases involve the blood vessels. The phospholipids forming the cell membranes of blood vessels have polyunsaturated fatty acids, mainly arachidonic acid, eicosapentaenoic acid, and docosahexaenoic acid bonded with them as component fatty acids. However, these polyunsaturated fatty acids cannot be synthesized *de novo* in animals and have to be directly or indirectly (in the case of arachidonic acid, by the precursor linoleic acid and in the case of eicosapentaenoic acid and docosahexaenoic acid, by the precursor α-linolenic acid) ingested from the diet. Up until now, polyunsaturated fatty acids, including linoleic acid and α-linolenic acid, have been noted for their action in reducing the cholesterol in the blood and for the expected effect of prevention of arteriosclerosis (*Shokuryo Eiyo Kenko (Food, Nutrition, and Health),* 72-78, 1991).

However, on the other hand, over-intake of the n-6-type polyunsaturated fatty acid linoleic acid reportedly conversely aggravates arterosclerosis *(Rinsho Eiyo (Clinical Nutrition)* 87, 254-259, 1995). For prevention of arterosclerosis, positive intake of the n-3-type polyunsaturated fatty acids α-linolenic acid, eicosapentaenoic acid, and docosahexaenoic acid is being recommended. Further, as pharmaceuticals as well, eicosapentaenoic acid ethyl ester is being marketed as a drug for treatment of hyperlipemia and arterosclerosis obliterans, but this mechanism is based on the action of reducing the cholesterol and not action on the cell membranes of blood vessels.

Therefore, there has been a strong demand for development of a compound preventing or alleviating symptoms or diseases due to aging of the blood vessels by preventing aging of the blood vessels and maintaining the elasticity of the blood vessels themselves, superior in application to food, and having few side effects.

### SUMMARY OF THE INVENTION

An object of the present invention is to provide a composition having arachidonic acid or a compound having arachidonic acid as a component fatty acid as an active ingredient and having an action preventing or alleviating symptoms or diseases due to aging of the blood vessels, a food or beverage having an action preventing or alleviating symptoms or diseases due to aging of the blood vessels, and a method of production of the same.

More specifically, the inventors attempted to provide a composition having at least one of arachidonic acid, an alcohol ester of an arachidonic acid, a triglyceride having arachidonic acid as a component fatty acid, and a phospholipid as an active ingredient and having an action preventing or alleviating a drop in elasticity of the blood vessels, arteriosclerosis (for example, ischemic cardiac diseases (myocardial infarction and angina), or cerebral apoplexy (cerebral hemorrhage and cerebral infarction), a food or beverage having a preventative or alleviating action, and a method of production of the same.

The inventors engaged in intensive research with the object of clarifying the effect of preventing or alleviating symptoms or diseases due to aging of the blood vessels by a composition having arachidonic acid or a compound having arachidonic acid as a component fatty acid as an active ingredient. As a result, surprisingly, when using old rats over 20-months old for tests for confirming the blood vessel relaxation action, they clarified the effect of the active ingredient of the present invention. Further, they succeeded in the industrial production of a triglyceride containing at least 20% of arachidonic acid produced by a microorganism, were able to use this for a test of the effects of the present invention, and clarified the effects. Further, they succeeded in the production of an oil containing a triglyceride with a medium-chain fatty acid bonded to the 1,3-position and with arachidonic acid bonded to the 2-position by the enzymatic method, were able to use this for test of the effects of the present invention, and clarified the effects.

Therefore, according to the present invention, there are provided a composition having arachidonic acid or a compound having arachidonic acid as a component fatty acid as an active ingredient and having an action preventing or alleviating symptoms or diseases due to aging of the blood vessels, a food or beverage having an action preventing or alleviating symptoms or diseases due to aging of the blood vessels, and a method of production of the same. More specifically, it is possible to provide a composition having at least one of arachidonic acid, an alcohol ester of an arachidonic acid, a triglyceride having arachidonic acid as a component fatty acid, and a phospholipid as an active ingredient and having an action preventing or alleviating a drop in elasticity of the blood vessels, arteriosclerosis (for example, ischemic cardiac diseases (myocardial infarction and angina), or cerebral apoplexy (cerebral hemorrhage and cerebral infarction), a food or beverage having a preventative or alleviating action, and a method of production of the same. This is particularly useful for people of contemporary society.

### BRIEF DESCRIPTION OF THE DRAWINGS

These and other objects and features of the present invention will become clearer from the following description of the preferred embodiments given with reference to the attached drawings, wherein:
FIG. 1 is a graph of the relationship between the acetylcholine concentration and blood vessel relaxation rate in an *in vitro* experiment using the blood vessels of old rats;
FIG. 2 is a graph of the effects of an arachidonic acid-containing diet with respect to an arachidonic acid content in thoracic arteries of old rats; and
FIG. 3 is a graph of the relationship between an arachidonic acid content in the blood vessels and a blood vessel relaxation rate in old rats.

### DESCRIPTION OF THE PREFERRED EMBODIMENTS

Preferred embodiments of the present invention will be described in detail below while referring to the attached figures.

The present invention relates to a composition having arachidonic acid or a compound having arachidonic acid as a component fatty acid as an active ingredient and having the action of preventing or alleviating symptoms or diseases due to aging of the blood vessels, a food or beverage having the action of preventing or alleviating symptoms or diseases due to aging of the blood vessels, and a method of production of the same. The symptoms or diseases due to aging of the blood vessels mean a drop in elasticity of the blood vessels, arteriosclerosis (for example ischemic cardiac diseases (myocardial infarction and angina) or cerebral apoplexy (cerebral hemorrhage and cerebral infarction), but the invention is not limited to these symptoms or diseases. All symptoms or diseases due to aging of the blood vessels are included.

P. Ghosh et al. report that if giving a high oil diet (20% oil content) to 12- to 14-week old female rats from 10 days before mating to 21 days after birth, even if returning to a normal diet (3% oil content) after weaning and raising them to 23 weeks age, the relaxation reaction due to the acetylcholine of the blood vessels (indicator of elasticity of blood vessels) becomes smaller compared with the group given the normal diet from day 10 before mating to day 21 after birth (J. *Physiol.* 533, 815-822, 2001).

Further, the fatty acid composition in the phospholipids of the blood vessels of rats born from parents of the high oil diet group showed a decrease in the amount of arachidonic acid compared with rats born from parents of the normal diet group even if raised on the normal diet after weaning. However, normal diet includes 0.13% of arachidonic acid, so under conventional thinking, the effects of arachidonic acid cannot be expected. The inventors took note of the drop in the blood vessel relaxation reaction of rats along with aging and discovered an effect of arachidonic acid overcoming this conventional thinking and thereby completed the present invention.

The active ingredient of the present invention is arachidonic acid. All compounds having arachidonic acid as a component fatty acid can be utilized. As compounds having arachidonic acid as a component fatty acid, arachidonates, for example a calcium salt and sodium salt, can be mentioned. Further, a lower alcohol ester of arachidonic acid, for example, arachidonic acid methyl ester, arachidonic acid ethyl ester, etc. may be mentioned. Further, triglyceride, phospholipid, sugar lipids, etc. having arachidonic acid as a component fatty acid may be utilized. Note that the present invention is not limited to the substances mentioned above. All compounds having arachidonic acid as a component fatty acid can be utilized.

When considering application to food, the arachidonic acid is preferably in the form of a triglyceride or phospholipid, in particular in the form of a triglyceride. There are almost no sources of supply of triglyceride containing arachidonic acid (synonymous with triglyceride where part or all of component fatty acids is comprised of arachidonic acid) in the natural world. The inventors made possible industrial utilization of triglyceride containing arachidonic acid as a component fatty acid for the first time and clarified the effect of the active ingredient of the present invention by evaluating the effect on blood vessel relaxation for old rats over 20 months age. They confirmed that there is the effect of preventing or alleviating the symptoms or diseases due to aging of the blood vessels.

Therefore, in the present invention, triglyceride comprising arachidonic acid-containing triglyceride in which part or all of the constituent fatty acid is arachidonic acid, serving as the active ingredient of the present invention, may be used. A triglyceride containing arachidonic acid is preferable when using an oil having a ratio of the arachidonic acid in the total fatty acids forming the triglyceride of at least 20 wt (W/W) %, preferably at least 30 wt%, more preferably at least 40 wt%, for food. Therefore, in the present invention, any oil obtained by culturing microorganisms having the ability to produce an oil (triglyceride) containing arachidonic acid may be used.

As microorganisms having the ability to produce an oil (triglyceride) containing arachidonic acid, microorganisms belonging to the *Mortierella, Conidiobolus, Pythium, Phytophthora, Penicillium, Cladosporium, Mucor, Fusarium, Aspergillus, Rhodotorula, Entomophthora, Echinosporangium,* and *Saprolegnia* may be mentioned.

In the microorganisms belonging to the subgenus *Mortierella* of the genus *Mortierella,* for example *Mortierella elongata, Mortierella exigua, Mortierella hygrophila, Mortierella alpina,* etc. may be mentioned. Specifically, *Mortierella elongata* IFO8570, *Mortierella* exigua IFO8571, *Mortierella hygrophila* IFO5941, *Mortierella alpina* IFO8568, ATCC16266, ATCC32221, ATCC42430, CBS219.35, CBS224.37, CBS250.53, CBS343.66, CBS527.72, CBS529.72, CBS608.70, CBS754.68, or other strains may be mentioned.

All of these strains can be obtained without any limitations from the Institute for Fermentation, Osaka (IFO), the American Type Culture Collection (ATCC), and the Centrralbureau voor Schimmelcultures (CBS). Further, it is also possible to use *Mortierella elongata* SAM0219 (Accession No. 8703 deposited at the Fermentation Research Institute) (Accession No. 1239 deposited at the Fermentation Research Institute) isolated by the research group of the present invention from the soil.

To incubate the strain used for the present invention, spores, mycelia or preincubation solutions obtained by incubation in advance are inoculated and incubated in a liquid medium or a solid medium. In the case of a liquid medium, as the carbon source, glucose, fructose, xylose, saccharose, maltose, soluble starch, molasses, glycerol, mannitol, or other generally used sources may be used, but the invention is not limited to these.

As the nitrogen source, peptone, yeast extract, malt extract, meat extract, Casamino acid, corn steep liquor, soybean protein, defatted soybeans, cottonseed waste, or other natural nitrogen sources and also urea and other organic nitrogen sources and further sodium nitrate, ammonium nitrate, ammonium sulfate, and other inorganic nitrogen sources may be used. In addition, in accordance with need, a phosphate, magnesium sulfate, iron sulfate, copper sulfate, or other inorganic salt and a vitamin etc. may be used as a minor nutrient. These medium ingredients are not particularly limited so long as they are in concentrations not obstructing growth of the microorganisms. In practice, in general, the carbon source should be a concentration of 0.1 to 40 wt%, preferably 1 to 25 wt%. The initial starting amount of nitrogen source added is 0.1 to 10 wt%, preferably 0.1 to 6 wt%. It is also possible to add a nitrogen source in the middle of the incubation.

Further, by controlling the medium carbon source concentration, it is also possible to use an oil (triglyceride) containing arachidonic acid in an amount of at least 45 wt (W/W) % as the active ingredient. The incubation is comprised of a cell growing period up to day 2 to day 4 of the incubation and an oil buildup period from day 2 to day 4 of the incubation. The initial starting carbon source concentration is made 1 to 8 wt%, preferably 1 to 4 wt%. The carbon source is sequentially added only in the cell growing period and the start of the oil buildup period. The total of the sequentially added carbon source is made 2 to 20 wt%, preferably 5 to 15 wt%. Note that the amount of sequential addition of the carbon source in the cell growing period and the start of the oil buildup period is controlled by addition in accordance with the initial starting nitrogen source concentration so that the carbon source concentration in the medium becomes zero from day 7 of incubation, preferably from day 6 of incubation, more preferably from day 4 of incubation, to thereby enable an oil (triglyceride) containing arachidonic acid in an amount of at least 45 wt% to be obtained. This can be used as the active ingredient of the present invention.

The incubation temperature of the arachidonic acid-producing microorganism depends on the microorganism used, but is 5 to 40°C, preferably 20 to 30°C. Further, after cells are grown by incubating at 20 to 30°C, the microorganism can continue to be incubated at 5 to 20°C to cause production of unsaturated fatty acids. By such temperature control as well, the ratio of the polyunsaturated fatty acids in the fatty acids produced can be raised. The pH of the medium is 4 to 10, preferably 5 to 9. Aerated stirred culture, shake culture, or static culture is performed. The incubation is usually performed for 2 to 30 days, preferably 5 to 20 days, more preferably 5 to 15 days.

Further, as a means to raise the ratio of the arachidonic acid in the oil (triglyceride) containing arachidonic acid, in addition to control of the medium carbon source concentration, it is possible to selectively hydrolyze the arachidonic acid-containing oil to obtain a high arachidonic acid-content oil. The lipase used for this selective hydrolysis has no position specificity of the triglyceride and falls in hydrolysis activity compared with the number of double bonds, so ester bonds of the fatty acids other than the polyunsaturated fatty acids are hydrolyzed. Further, an ester exchange reaction occurs between the PUFA partial glycerides produced, resulting in triglyceride with enhanced polyunsaturated fatty acids ("Enhancement of Archidonic: Selective Hydrolysis of a Single-Cell Oil from *Mortierella* with *Candida cylindracea* Lipase": J. Am. *Oil Chem. Soc.,* 72, 1323-1327 (1998)).

In this way, it is possible to use the oil (triglyceride) containing a high concentration of arachidonic acid obtained by selective hydrolysis of the arachidonic acid-containing oil as the active ingredient of the present invention. The ratio of the arachidonic acid to the total fatty acids of the oil (triglyceride) containing arachidonic acid of the present invention is preferably high for the purpose of eliminating the effects of other fatty acids, but the invention is not limited to a high ratio. In actuality, in the case of application to food, the absolute amount of the arachidonic acid sometimes becomes a problem. Use is substantially possible even with an oil (triglyceride) containing 10 wt% or more of arachidonic acid.

Further, as the triglyceride where part or all of the component fatty acids is comprised of arachidonic acid, triglyceride with a medium-chain fatty acid bonded to the 1,3-position and with arachidonic acid bonded to the 2-position may be used. Further, an oil (triglyceride) including triglyceride with a medium-chain fatty acid bonded to the 1,3-position and with arachidonic acid bonded to the 2-position in an amount of at least 5 mol%, preferably at least 10 mol%, more preferably at least 20 mol%, and most preferably at least 30 mol%, may be used. The medium-chain fatty acid bonded to the 1,3-position of the triglyceride used may be one selected from fatty acids having 6 to 12 carbon atoms. As fatty acids having 6 to 12 carbon atoms, for example, caprylic acid or capric acid etc. may be mentioned. In particular, 1,3-capryloyl-2-arachidonoyl-glycerol (hereinafter also referred to as "8A8") is preferable.

Such a triglyceride with a medium-chain fatty acid bonded to the 1,3-position and with arachidonic acid bonded to the 2-position is the optimal oil (triglyceride) when dealing with senior citizens. In general, when oil (triglyceride) is ingested and enters the small intestine, it is hydrolyzed by the pancreatic lipase, but this pancreatic lipase is 1,3-position specific. The 1,3-position of the triglyceride is cleaved to create two molecules of free fatty acid. Simultaneously, one molecule of 2-monoacyl glycerol (2-MG) is produced. This 2-MG is extremely high solubility in bile acid and good in absorption, so in general 2-position fatty acid is said to be good in absorption. Further, 2-MG acts like a surfactant when dissolved in bile acid and acts to raise the absorption of free fatty acids.

Next, the free fatty acids and 2-MG produce bile acid complex micelles together with cholesterol or phospholipids etc. These are taken into the small intestine epithelial cells where rebonding of triacyl glycerol occurs. Finally, the result is released to the lympha as chylomicron. However, looking at the fatty acid characteristics of this pancreatic lipase, this is high in saturated fatty acids and the arachidonic acid is hard to cleave. A further problem is that the pancreatic lipase activity falls along with aging, so for senior citizens susceptible to symptoms and diseases due to aging of the blood vessels, a triglyceride with a medium-chain fatty acid bonded to the 1,3-position and with arachidonic acid bonded to the 2-position is the optimal oil (triglyceride).

As one specific method of production of a triglyceride with a medium-chain fatty acid bonded to the 1,3-position and with arachidonic acid bonded to the 2-position, it is possible to cause the action of lipase which acts only on the ester bond of the 1,3-position of the triglyceride in the presence of an oil (triglyceride) containing arachidonic acid and a medium-chain fatty acid. The oil (triglyceride) used as a material is a triglyceride having arachidonic acid as a component fatty acid. When the ratio of the arachidonic acid with respect to the total fatty acids forming the triglyceride is high, the drop in the reaction yield due to the increase of the unreacted oil (material triglyceride and triglyceride with only one of fatty acids at 1,3-position formed by a medium-chain fatty acid) is prevented by making the temperature 30 to 50°C, preferably 40 to 50°C, higher than the ordinary enzyme reaction temperature of 20 to 30°C.

As the lipase acting specifically on the ester bond of the 1,3-position of the triglyceride, for example, one produced by a microorganism such as the genus *Rhizopus, Rhizomucor,* and *Aspergillus,* porcine pancreatic lipase, etc. may be mentioned. For this lipase, a commercial one can be used. For example, the lipase of *Rhizopus delemar* (made by Tanabe Seiyaku, Talipase ®), the lipase of *Rhizomucor miehei* (made by Novo Nordisk, Lipozyme ® IM), the lipase of *Aspergillus niger* (made by Amano Seiyaku, Lipase A ®), etc. may be mentioned, but the invention is not limited to these enzymes. It is possible to use all 1,3-position-specific lipases.

As to the mode of use of the lipase, since the reaction temperature is made at least 30°C, preferably at least 40°C, for the purpose of raising the reaction efficiency, it is preferable to use a lipase immobilized at an immobilizing carrier for the purpose of giving heat resistance to the enzyme. As the immobilizing carrier, a porous (high porous) resin type ion exchange resin carrier having a pore size of at least about 100 Angstroms, for example, Dowex MARATHON WBA, may be mentioned. However, the invention is not limited to these immobilizing carriers. It is possible to use all immobilizing carriers able to give heat resistance.

1,3-position-specific lipase is suspended in a 0.5-to 20-fold amount of an aqueous solution with respect to the immobilizing carrier. A 2- to 5-fold amount of cold acetone (for example, -80°C) is gradually added to the suspension while stirring to form a precipitate. This precipitate may be dried in vacuo to prepare an immobilized enzyme. Further, a simple method may be used to dissolve a 0.05- to 0.4-fold amount of 1,3-position-specific lipase with respect to the immobilizing carrier in the minimum amount of water, mix in the immobilizing carrier while stirring, and dry the result in vacuo to prepare the immobilized enzyme. By this operation, approximately 90% of the lipase is immobilized at the carrier, but with this as it is, no ester exchange activity is exhibited at all. By pretreatment in a substrate to which 1 to 10 wt (w/v) % water is added, preferably in a substrate to which 1 to 3 wt% of water is added, the immobilizing enzyme can be efficiently activated and used for production.

Depending on the type of the enzyme, the amount of moisture added to the reaction system is extremely important. When water is not included, the ester exchange proceeds with difficulty. Further, when the amount of moisture is too great, hydrolysis occurs and the rate of recovery of the glyceride falls (if hydrolysis occurs, diglyceride and monoglyceride are produced). However, in this case, by using an immobilized enzyme activated by pretreatment, the amount of moisture added to the reaction system becomes no longer important and an efficient ester exchange reaction can be caused even in a system not containing any water at all. Further, by selecting the type of the enzyme agent, it is also possible to omit the pretreatment.

In this way, by using an immobilized enzyme having heat resistance and raising the enzyme reaction temperature, even in an oil (triglyceride) containing arachidonic acid with a low reactivity to the 1,3-position-specific lipase, the reaction efficiency is not caused to fall and triglyceride (8A8) with a medium-chain fatty acid bonded to the 1,3-position and with arachidonic acid bonded to the 2-position can be efficiently produced.

In the method of production of a food or beverage having the action of preventing or alleviating symptoms or diseases due to aging of the blood vessels, it is possible to blend in arachidonic acid and/or a compound having arachidonic acid as a component fatty acid alone or together with a food or beverage ingredient in which arachidonic acid is substantially not contained or is contained in a slight amount. Here, the "slight amount" means an amount where even if the food or beverage ingredient contains arachidonic acid and even if a food composition containing this is ingested by a person, the daily arachidonic acid intake of the present invention explained later is not reached.

In particular, in the case of a triglyceride where part or all of the component fatty acids consists of arachidonic acid, there are unlimited possibilities for the applications of the oil (triglyceride). Use as materials or additives of food, beverages, cosmetics, and pharmaceuticals is possible. The objectives of use and the amounts of use are not limited in any way.

For example, as food compositions, general foods, functional food, nutritional supplements, food for specified health uses, milk formula for premature infants, milk formula for infants, baby food, food for pregnant women and new mothers, and food for senior citizens may be mentioned. As examples of food containing oil, meat, fish, nuts, or other natural food inherently containing oil, soups and other food to which oil is added at the time of preparation, donuts and other food using oil as a heat medium for cooking, butter and other oil-type food, cookies and other processed food to which oil is added at the time of processing, hard biscuits and other food sprayed or coated with oil at the time of finishing, etc. may be mentioned. Further, the composition of the invention may be added to farm produce, fermented food, animal produce, marine produce, and beverages. Further, either the form of functional food or pharmaceuticals is possible. For example, an enteric nutrient, powder, granules, troche, internal liquid medicines, suspensions, emulsions, syrups, and other processed forms are possible.

Further, the composition of the present invention may include, in addition to the active ingredients of the present invention, the various carriers and additives generally used for food and beverages, pharmaceuticals, and quasi-pharmaceuticals. It preferably includes an antioxidant for the purpose of preventing oxidation of the active ingredient of the present invention. As antioxidants, for example, tocopherols, flavone derivatives, phyllodulcin, kojic acid, gallic acid derivatives, catechins, fukiic acid, gossypol, pyrazine derivatives, sesamol, guaiol, guaiacic acid, p-cumaric acid, nordihydroguaiaretic acid, sterols, terpenes, nucleic acid bases, carotinoids, lignans, or other natural antioxidants and ascorbic acid palmitic acid ester, ascorbic acid stearic acid ester, butyl hydroxyanisole (BHA), butyl hydroxytoluene (BHT), mono-t-butylphenol (HMBP), and other synthetic antioxidants may be mentioned.

In the tocopherols, α-tocopherol, β-tocopherol, γ-tocopherol, δ-tocopherol, ε-tocopherol, ζ-tocopherol, η-tocopherol, and tocopherol ester (tocopherol acetate etc.) and further tocotriethanol as a similar compound may be mentioned. Further, in carotinoids, for example, β-carotin, cantaxanthin, astaxanthin, etc. may be mentioned.

The composition of the present invention may include, in addition to the active ingredient of the present invention, as a carrier various carriers, extenders, diluents, thickners, dispersants, excipients, binder solvents (for example, water, ethanol, vegetable oil), dissolution aids, buffers, dissolution accelerators, gel agents, suspension agents, flour, rice powder, starch, corn starch, polysaccharides, milk protein, collagen, rice oil, lecithin, etc. As additives, for example, vitamins, sweeteners, organic acids, coloring agents, flavors, anti-moisturizing agents, fiber, electrolytes, minerals, nutrients, antioxidants, preservatives, perfumes, humectants, natural plant extracts, vegetable extracts, etc. may be mentioned, but the invention is not limited to these.

The main active ingredient of arachidonic acid and a compound having arachidonic acid as a component fatty acid is arachidonic acid. The daily intake of arachidonic acid from diet is reportedly 0.14 g in the Kanto region of Japan and 0.19 to 0.20 g in the Kansai region *(Shishitsu Eiyogaku (Lipid Nutrition)* 4, 73-82, 1995). In view of the fact that the intake of oil of senior citizens falls, the fact that the pancreatic lipase activity falls, etc., it is necessary to ingest arachidonic acid in considerable amounts or even more. Therefore, the adult (for example, body weight of 60 kg) daily intake of the arachidonic acid and a compound having arachidonic acid as a component fatty acid of the present invention is made, converted to amount of arachidonic acid, 0.001 to 20 g, preferably 0.01 to 10 g, more preferably 0.05 to 5 g, most preferably 0.1 to 2 g.

When actually applying the active ingredient of the present invention to a food or beverage, the absolute amount of the arachidonic acid blended in the food is also important. However, even the absolute amount blended into the food or beverage changes depending on the intake of the food or beverage into which it is mixed, so when mixing a triglyceride containing a triglyceride in which part or all of the component fatty acids is comprised of arachidonic acid into food, it is preferably mixed in to give at least 0.001 wt% as arachidonic acid, preferably at least 0.01 wt%, more preferably at least 0.1 wt%. Further, when mixing a triglyceride with a medium-chain fatty acid bonded to the 1,3-position and arachidonic acid bonded to the 2-position into a food or beverage, it is preferably mixed in to give at least 0.0003 wt%, preferably at least 0.003 wt%, more preferably at least 0.03 wt%.

When using the composition of the present invention as a pharmaceutical, it may be produced by commonly used methods in the field of drug-making, for example, the methods described in the Japanese Pharmacoepea or methods based on the same. Further, when using the composition of the present invention as a pharmaceutical, the amount of the active ingredient contained in the composition is not particularly limited so long as the object of the present invention is achieved. Use is possible in an suitable and appropriate ratio.

When using the composition of the present invention as a pharmaceutical, it is preferably administered in the form of units of administration, preferably oral administration. The dosage of the composition of the present invention differs according to the age, body weight, symptoms, number of doses, etc., but for example the arachidonic acid and/or compound having arachidonic acid as a component fatty acid of the present invention should be administered, per adult (assuming about 60 kg) per day, usually about 0.001 g to 20 g, preferably 0.01 g to 10 g, more preferably 0.05 to 5 g, most preferably 0.1 g to 2 g, converted to amount of arachidonic acid, divided into one to three doses per day.

The phospholipids forming the cell membranes of the blood vessels have high unsaturated fatty acids, mainly arachidonic acid, eicopentaenoic acid, and docasahexaenoic acid, as component fatty acids bonded to them. If considering a balance, a combination with eicopentaenoic acid and/or docasahexaenoic acid is preferable. Further, a food or beverage where the ratio of the arachidonic acid/eicopentaenoic acid and/or docasahexaenoic acid in the combination of arachidonic acid and docasahexaenoic acid is in the range of 0.25 to 8 is most preferred.

### Examples

Next, the present invention will be explained in more detail by examples. However, the present invention is not limited to these examples.

### Reference Example 1. Method of Production of Triglyceride Having Arachidonic Acid as Component Fatty Acid

As the arachidonic acid-producing microorganism, *Mortierella alipina* CBS754.68 was used. 6 kl of a medium containing 1.8% of glucose, 3.1% of defatted soybean powder, 0.1% of soybean oil, 0.3% of KH₂PO₄, 0.1% of Na₂SO₄, 0.05% of CaCl₂-2H₂O, and 0.05% of MgCl₂-6H₂O was prepared in a 10 kl incubation tank. The initial starting pH was adjusted to 6.0. 30 liters of the preincubation solution was inoculated and aerated stirred culture was performed under conditions of a temperature of 26°C, aeration of 360 m³/h, and a tank internal pressure of 200 kPa for 8 days. Note that the stirring speed was adjusted so as to maintain the solute oxygen concentration at 10 to 15 ppm. Further, the glucose concentration is controlled by the fed-batch method so that the glucose concentration in the medium is in the range of 1 to 2.5% up to day 4, then is 0.5 to 1% (the above % means weight (W/V)%).

After the end of incubation, filtration and drying were used to recover cells containing triglyceride having arachidonic acid as a component fatty acid. The obtained cells were converted to an oil by hexane extraction. After a purification process for edible oil (degumming, deoxidation, deodorization, and decolorization), 150 kg of arachidonic acid-containing triglyceride (triglyceride comprising arachidonic acid-containing triglyceride in which part or all of the constituent fatty acid is arachidonic acid) was obtained. The obtained oil (triglyceride) was methyl-esterified and the obtained fatty acid methyl ester was analyzed by gas chromatography, whereupon the ratio of the arachidonic acid in the total fatty acids was found to be 40.84%.

Note that the contents of palmitic acid, stearic acid, oleic acid, linoleic acid, γ-linolenic acid, dihomo-γ-linolenic acid, etc. were 11.63%, 7.45%, 7.73%, 9.14%, 2.23%, and 3.27%. Further, the above arachidonic acid-containing oil (triglyceride) was ethyl-esterified and 99% arachidonic acid ethyl ester was isolated and purified by the established method of high pressure liquid chromatography from the fatty acid ethyl ester mixture containing 40% of arachidonic acid ethyl ester.

### Reference Example 2. Production of Triglyceride Containing at Least 5% Triglyceride (8A8) Having medium-Chain Fatty Acid Bonded to 1,3-Position and Arachidonic Acid Bonded to 2-Position

100 g of an ion exchange resin carrier (Dowex MARATHON WBA: Dow Chemical) was suspended in 80 ml of a *Rhizopus delemar* lipase aqueous solution (Talipase® powder, 12.5%: Tanabe Seiyaku), 240 ml of cold acetone (-80°C) was stirred in, and the mixture was dried in vacuo to obtain immobilized lipase.

Next, 80 g of triglyceride containing 40% of arachidonic acid obtained at Reference Example 1 (SUNTGA40S), 160 g of caprylic acid, 12 g of the above immobilized lipase, and 4.8 ml of water were reacted at 30°C for 48 hours while stirring (130 rpm). After the end of the reaction, the reaction solution was removed to obtained the activated immobilized enzyme.

Next, 10 g of immobilized lipase *(Rhizopus delemar* lipase, carrier: Dowex MARATHON WBA) was packed into a jacketed glass column (1.8 x 12.5 cm, volume 31.8 ml). A reaction oil comprised of a 1:2 mixture of the SUNTGA40S obtained in Reference Example 1 and caprylic acid was passed through the column at a fixed flow rate (4 ml/h) for a continuous reaction, whereupon 400 g of the reaction oil was obtained. Note that the column temperature was made 40 to 41°C. The unreacted caprylic acid and free fatty acids were removed from the obtained reaction oil by molecular distillation and the result was passed through a purification process for edible oil (degumming, deoxidation, deodorization, and decolorization) to obtain an oil containing 8A8 (triglyceride).

Further, gas chromatography and high pressure liquid chromatography were used to investigate the ratio of 8A8 in the obtained 8A8-containing oil (triglyceride), whereupon the ratio was found to be 31.6% (note that the ratios of 8P8, 808, 8L8, 8G8, and 8D8 were 0.6, 7.9, 15.1, 5,2, and 4.8%. The fatty acids P, O, L, G, and D bonded to the 2-position of the triglyceride are palmitic acid, oleic acid, linoleic acid, γ-linolenic acid, and dihomo-γ-linolenic acid and 8P8 mean 1,3-capryloyl-2-palmitolein-glycerol, 808 1,3-capryloyl-2-oleoyl-glycerol, 8L8 1,3-capryloyl-2-linoleoyl-glycerol, 8G8 1,3-capryloyl-2-γ-linolenoyl-glycerol, and 8D8 1,3-capryloyl-2-dihomo-γ-linolenoyl-glycerol). Note that 96 mol% 8A8 was isolated and purified by the established method of high pressure liquid chromatography from the obtained 8A8-containing oil (triglyceride).

### Example 1. Evaluation of SUNTGA40S Due to Acetylcholine Blood Vessel Relaxation Reaction Test

The effect of triglyceride (arachidonic acid-containing oil (SUNTGA40S)) having arachidonic acid as a component fatty acid prepared in Reference Example 1 on the acetylcholine blood vessel relaxation reaction was investigated using rats. As the test groups of old rats, 15 19-month old male Fischer rats were divided into a control diet group (8 rats: OC group) and a SUNTGA40S compound diet group (7 rats: OA group). The groups were given the control diet and the SUNTGA40S diet shown in Table 1. Further, using young rats as a control group, 10 1-month old male Fischer rats were given the control diet shown in Table 1 (10 rats: YC group).

**Table 1. Experiment Diet**

| | Control diet (g/kg) | SUNTGA40S diet (g/kg) |
|---|---|---|
| Casein | 200 | 200 |
| DL-methione | 3 | 3 |
| Corn starch | 150 | 105 |
| Sucrose | 500 | 500 |
| Cellulose powder | 50 | 50 |
| Corn oil | 50 | 45 |
| Mineral AlN-76 | 35 | 35 |
| Vitamin AlN-76 | 10 | 10 |
| Choline bitartarate | 2 | 2 |
| Vitamin E | 0.05 | 0.05 |
| SUNGA40S0 | 0 | 5 |

The amount of intake of rats was about 20 g and the daily intake of SUNTGA40S per rat was 100 mg. Of the total fatty acids bonded with the arachidonic acid-containing oil (SUNTGA40S) prepared in Reference Example 1, 40% is arachidonic acid, so the daily intake of arachidonic acid per rat became 40 mg. This 40 mg corresponds to 133 mg/60 kg/day converted to human intake.

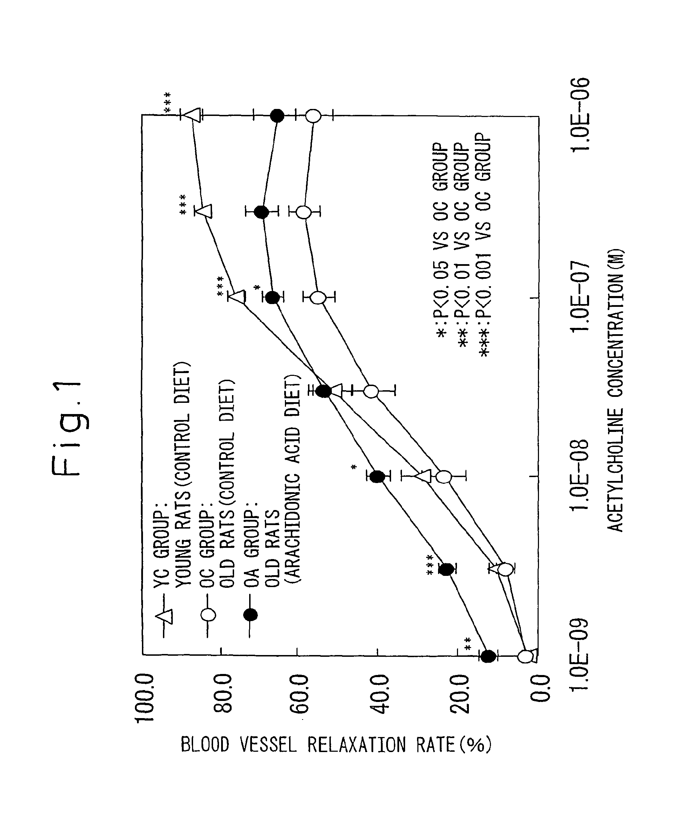

In the third month of raising (in the case of old rats, 22-month age and in the case of young rats, four-month age), the thoracic arteries were excised. Part was used for the *in vitro* blood vessel relaxation reaction test, while the remaining blood vessels were used for analysis of the fatty acid composition. The blood vessel relaxation reaction test called for preparing blood vessel rings using the thoracic arteries carefully excised so as not to damage the endothelium of the blood vessels, causing preshrinkage by phenylephrine, then measuring the relaxation reaction by 10⁻⁹M to 10⁻⁶M concentration acetylchlorine. As a result, compared with young rats, the relaxation reaction of the blood vessels of old rats clearly dropped, but the intake of arachidonic acid-containing oil improved the blood vessel relaxation reaction of old rats (FIG. 1).

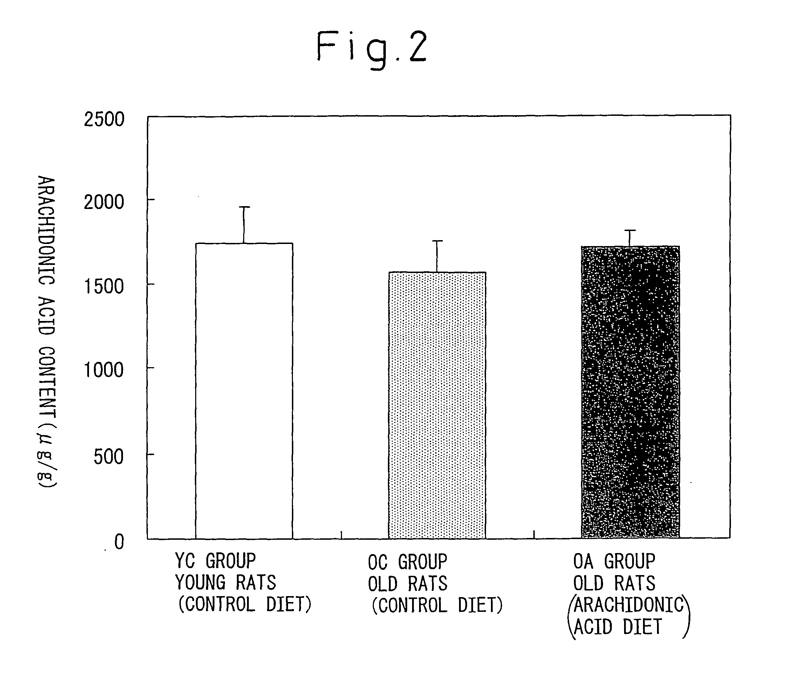

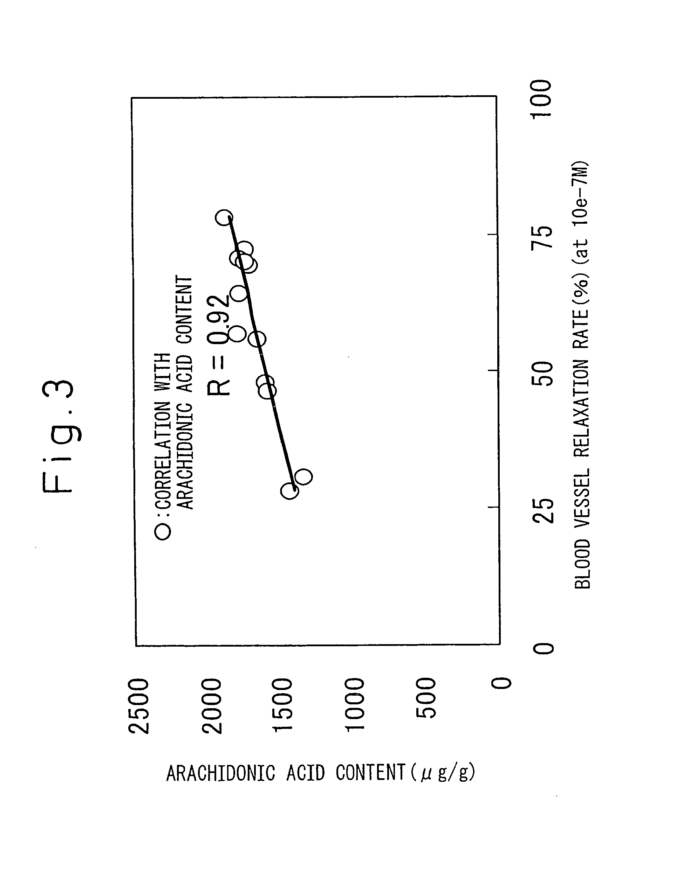

Next, the total lipids were extracted by the Folch method from the excised thoracic arteries. Water was removed from the extracted lipid as an azeotrope with ethanol, then the fatty acid composition was analyzed by gas chromatography by 10% hydrochloric acid-methanol as a fatty acid methyl ester. As a result, compared with young rats, the amount of arachidonic acid per unit weight of blood vessels of old rats clearly dropped, but due to the intake of arachidonic acid-containing oil, the amount of arachidonic acid per unit weight of blood vessels of old rats increased (FIG. 2). Further, the correlation between the arachidonic acid content per unit weight of thoracic artery and the blood vessel relaxation rate due to 10⁻⁷M of acetylcholine in old rats was sought. As a result, a significant, high correlation (R=0.92) was recognized with the arachidonic acid content (FIG. 3). In this way, it was shown for the first time that by ingesting an arachidonic acid-containing oil, the blood vessel relaxation ability or the blood vessel endothelium cell functions. It was clarified for the first time that this effect was due to the arachidonic acid.

### Example 2. Evaluation of 8A8 (96 mol%) by Acetylcholine Blood Vessel Relaxation Reaction Test

The effect of triglyceride (8A8) with a medium-chain fatty acid bonded to the 1,3-position and with arachidonic acid bonded to the 2-position prepared in Reference Example 2 on the acetylcholine blood vessel relaxation reaction was investigated using rats. As the test groups of old rats, 15 19-month old male Fischer rats were divided into a control diet group (8 rats: OC group) and an 8A8 diet group (7 rats: OB group). The groups were given the control diet and the 8A8 diet shown in Table 2. Further, using young rats as a control group, 10 1-month old male Fischer rats were given the control diet shown in Table 2 (10 rats: YC group). Note that for the 8A8 used for the 8A8 diet, 96 mol% 8A8 obtained in Reference Example 2 was used.

**Table 2. Experiment Diet**

| | Control diet (g/kg) | 8A8 diet (g/kg) |
|---|---|---|
| Casein | 200 | 200 |
| DL-methione | 3 | 3 |
| Corn starch | 150 | 150 |
| Sucrose | 500 | 500 |
| Cellulose powder | 50 | 50 |
| Corn oil | 50 | 45 |
| Mineral AlN-76 | 35 | 35 |
| Vitamin AlN-76 | 10 | 10 |
| Choline bitartarate | 2 | 2 |
| Vitamin E | 0.05 | 0.05 |
| 8A8 | 0 | 4.2 |

The amount of intake of rats was about 20 g and the molecular weight of 8A8 was 628.7, so the experiment diet was designed so that the daily arachidonic acid intake per rat of the 8A8 diet group became 40 mg. This 40 mg corresponds to 133 mg/60 kg/day converted to human intake.

In the third month of raising (in the case of old rats, 22-month age and in the case of young rats, four-month age), the thoracic arteries were excised. Part was used for the *in vitro* blood vessel relaxation reaction test, while the remaining blood vessels were used for analysis of the fatty acid composition. The blood vessel relaxation reaction test called for preparing blood vessel rings using the thoracic arteries carefully excised so as not to damage the endothelium of the blood vessels, causing preshrinkage by phenylephrine, then measuring the relaxation reaction by 10⁻⁹M to 10⁻⁶M concentration acetylchlorine.

As a result, the averages of the blood vessel relaxation rates by acetylcholine 10⁻⁷M were 80.5% for young rats ingesting the control diet, 52.3% for old rats ingesting the control diet, and 62.7% for old rats ingesting the 8A8 diet. The intake of 8A8 improved the blood vessel relaxation response of old rats.

### Example 3. Preparation of Oil (Triglyceride)-Containing Capsules Having Arachidonic Acid as Component Fatty Acid

Water was added to 100 parts by weight of gelatin and 35 parts by weight of food additive glycerin to dissolve them at 50 to 60°C and prepare a gelatin coating with a viscosity of 2000 cp. Next, 0.05 wt% of vitamin E oil was mixed with the arachidonic acid-containing oil (triglyceride) obtained in Reference Example 1 to prepare the Content 1. 0.05 wt% of vitamin E was blended with an oil containing 32 mol% of the 8A8 obtained at Reference Example 2 to prepare the Content 2.

Next, 50 wt% of the arachidonic acid-containing oil (triglyceride) obtained at Reference Example 1 and 50 wt% of fish oil (tuna oil: ratios of eicosapentaenoic acid and docasahexaenoic acid in total fatty acids respectively 5.1% and 26.5%) were mixed and 0.05 wt% of vitamin E oil was mixed in to prepare the Content 3. 80 wt% of the arachidonic acid-containing oil (triglyceride) and 20 wt% of fish oil (tuna oil: ratios of eicosapentaenoic acid and docasahexaenoic acid in total fatty acids respectively 5.1% and 26.5%) were mixed and 0.05 wt% of vitamin E oil was mixed in to prepare the Content 4. To the 99% arachidonic acid ethyl ester obtained in Reference Example 1, 0.05 wt% of vitamin E oil was mixed to prepare the Content 5. Using the Contents 1 to 5, capsules were formed and dried by ordinary methods to produce soft capsules containing 200 mg of content per capsule.

### Example 4. Use in Fat Infusion

400 g of an oil (triglyceride) containing 32% of 8A8 obtained in Reference Example 2, 48 g of purified egg yolk lecithin, 20 g of oleic acid, 100 g of glycerin, and 40 ml of 0.1N caustic soda were added and dispersed by a homogenizer, then injection use distilled water was added to dilute this to 4 liters. The result was emulsified by a high pressure spray type emulsifier to prepare a lipid emulsion. The lipid emulsion was injected into plastic bags 200 ml at a time, then was sterilized by high pressure steam at 121°C for 20 minutes to obtain a fat infusion.

### Example 5. Use in Juice

2 g of β-cyclodextrin was added to 20 ml of a 20% ethanol aqueous solution. While stirring this by a stirrer, 100 mg of the arachidonic acid-containing triglyceride obtained in Reference Example 1 (containing 0.05 wt% of vitamin E) was added and the result was incubated at 50°C for 2 hours. The mixture was cooled to room temperature (about 1 hour), then further incubated, while continuing stirring, at 4°C for 10 hours. The precipitate produced was recovered by centrifugal separation, washed by n-hexane, then freeze-dried to obtain 1.8 g of a cyclodextrin-enveloped compound containing an arachidonic acid-containing triglyceride. 1 g of this powder was uniformly mixed with 10 liters of juice to prepare juice containing arachidonic acid-containing triglyceride.

While the invention has been described with reference to specific embodiments chosen for purpose of illustration, it should be apparent that numerous modifications could be made thereto by those skilled in the art without departing from the basic concept and scope of the invention.

## Claims

1. A composition having an action preventing or alleviating symptoms or diseases due to aging of blood vessels, including any one of arachidonic acid and a compound having arachidonic acid as a component fatty acid.

2. A composition as set forth in claim 1, wherein said compound having arachidonic acid as a component fatty acid is any one of an alcohol ester of arachidonic acid, a triglyceride having arachidonic acid as a component fatty acid, and a phospholipid.

3. A composition as set forth in claim 2, wherein in said triglyceride having arachidonic acid as a component fatty acid, the ratio of the arachidonic acid in the total fatty acids comprising the triglyceride is at least 20%.

4. A composition as set forth in claim 2, wherein said triglyceride having arachidonic acid as a component fatty acid is extracted from a microorganism belonging to any one of the *Mortierella, Conidiobolus, Pythium, Phytophthora, Penicillium, Cladosporium, Mucor, Fusarium, Aspergillus, Rhodotorula, Entomophthora, Echinosporangium,* and *Saprolegnia.*

5. A composition as set forth in claim 2, wherein said triglyceride having arachidonic acid as a component fatty acid is a triglyceride including at least 5% of a triglyceride with a medium-chain fatty acid bonded to the 1,3-position and with arachidonic acid bonded to the 2-position.

6. A composition as set forth in claim 5, wherein said medium-chain fatty acid is selected from a fatty acid having 6 to 12 carbon atoms.

7. A composition as set forth in any one of claims 1 to 6, wherein said symptom due to aging of the blood vessels is a drop in the elasticity of the blood vessels.

8. A composition as set forth in any one of claims 1 to 6, wherein said symptom due to aging of the blood vessels is arteriosclerosis and the disease is an ischemic cardiac disease.

9. A composition as set forth in claim 8, wherein said ischemic cardiac disease is one of myocardial infarction and angina.

10. A composition as set forth in any one of claims 1 to 6, wherein said symptom due to aging of the blood vessels is arteriosclerosis and the disease is cerebral apoplexy.

11. A composition as set forth in claim 10, wherein said cerebral apoplexy is one of cerebral hemorrhage and cerebral infarction.

12. A food or beverage having an action preventing or alleviating symptoms or diseases due to aging of blood vessels, including any one of arachidonic acid and a compound having arachidonic acid as a component fatty acid.

13. A food or beverage as set forth in claim 12, wherein said compound having arachidonic acid as a component fatty acid is any one of an alcohol ester of arachidonic acid, a triglyceride having arachidonic acid as a component fatty acid, and a phospholipid.

14. A food or beverage as set forth in claim 13, wherein in said triglyceride having arachidonic acid as a component fatty acid, the ratio of the arachidonic acid in the total fatty acids comprising the triglyceride is at least 20%.

15. A food or beverage as set forth in claim 13, wherein said triglyceride having arachidonic acid as a component fatty acid is extracted from a microorganism belonging to any one of the *Mortierella, Conidiobolus, Pythium, Phytophthora, Penicillium, Cladosporium, Mucor, Fusarium, Aspergillus, Rhodotorula, Entomophthora, Echinosporangium,* and *Saprolegnia.*

16. A food or beverage as set forth in claim 13, wherein said triglyceride having arachidonic acid as a component fatty acid is a triglyceride including at least 5% of a triglyceride with a medium-chain fatty acid bonded to the 1,3-position and with arachidonic acid bonded to the 2-position.

17. A food or beverage as set forth in claim 16, wherein said medium-chain fatty acid is selected from a fatty acid having 6 to 12 carbon atoms.

18. A food or beverage having an action preventing or alleviating symptoms or diseases due to aging of the blood vessels as set forth in claims 12 to 17 wherein said a food or beverage is any one of a functional food, a nutritional supplement, a food for specified health uses, and geriatric food.

19. A food or beverage as set forth in any one of claims 12 to 17, wherein said symptom due to aging of the blood vessels is a drop in the elasticity of the blood vessels.

20. A food or beverage as set forth in any one of claims 12 to 17, wherein said symptom due to aging of the blood vessels is arteriosclerosis and the disease is an ischemic cardiac disease.

21. A food or beverage as set forth in claim 20, wherein said ischemic cardiac disease is one of myocardial infarction and angina.

22. A food or beverage as set forth in any one of claims 12 to 17, wherein said symptom due to aging of the blood vessels is arteriosclerosis and the disease is cerebral apoplexy.

23. A food or beverage as set forth in claim 22, wherein said cerebral apoplexy is one of cerebral hemorrhage and cerebral infarction.

24. A food or beverage comprised of a combination of arachidonic acid and at least one of eicosapentaenoic acid and docosahexaeonic acid wherein the ratio of the arachidonic acid and said at least one of eicosapentaenoic acid and docosahexaenoic acid is in the range of 0.25 to 8.

25. A food or beverage including at least 0.001% (weight) of a triglyceride with a medium-chain fatty acid bonded to the 1,3-position and with arachidonic acid bonded to the 2-position.

26. A food or beverage as set forth in claim 25, wherein said medium-chain fatty acid is selected from a fatty acid having 6 to 12 carbon atoms.

27. A method of producing a food or beverage having an action preventing or alleviating symptoms or diseases due to aging of the blood vessels, including blending in at least one of arachidonic acid and a compound having arachidonic acid as a component fatty acid alone or together with a food or beverage ingredient in which arachidonic acid is substantially not contained or is contained in a slight amount.

28. A method for prevention or treatment of symptoms or diseases due to aging of the blood vessels, including administering a subject requiring that administration one of arachidonic acid and a compound having arachidonic acid as a component fatty acid.
